# EUROPEAN PATENT SPECIFICATION

(11) **EP 0 841 945 B1**
(45) Date of publication and mention of the grant of the patent: **08.03.2006**
(21) Application number: 96926021.5
(22) Date of filing: 05.08.1996
(51) Int. Cl.: A61K 39/385

(54) **CELL DERIVED ANTIGEN PRESENTING VESICLES**
ANTIGEN PRESENTIERENDE BLÄSCHEN, DIE VON ZELLEN ABLEITEN
VESICULES DERIVEES DE CELLULES, PRESENTANT DES ANTIGENES

(30) Priority: 03.08.1995 EP 95202123
(43) Date of publication of application: 20.05.1998
(73) Proprietor: Rijksuniversiteit te Leiden, 2312 AV Leiden (NL); Universiteit Utrecht, 3584 CG Utrecht (NL)
(72) Inventor: GEUZE, Johannes, J., NL-3584 CG Utrecht (NL); MELIEF, Cornelis, J., M., NL-2312 AV Leiden (NL)
(74) Representative: Becker, Philippe
(86) International application number: PCT/NL1996/000317
(87) International publication number: WO 1997/005900

(56) References cited:
- NATURE, vol. 315, 1985, LONDON GB, pages 327-329, XP002016307 WALDEN P. ET AL: "Induction of regulatory T-lymphocyte responses by liposomes carrying major histocompatibility complex molecules and forein antigen"
- JOURNAL OF IMMUNOLOGY, vol. 151, no. 8, 1993, BALTIMORE US, pages 3988-3998, XP002016308 HARDING C.V. ET AL: "Immunogenic peptides bind to class II MHC molecules in an early lysosomal compartment"
- NATURE, vol. 369, 1994, LONDON GB, pages 113-120, XP002016309 AMIGORENA S. ET AL: "Transient accumulation of new class II MHC molecules in a novel endocytic compartment in B lymphocytes" cited in the application

## Description

The invention relates to the field of immunology, especially the cellular responses of the immune system, more in particular to the induction of said responses by peptides presented in the context of major histocompatibility complexes I and/or II.

It is known that antigen presenting cells take up antigens through endocytosis, whereafter these antigens are cleaved into peptides which are presented at the surface of said antigen presenting cells in the context of a major histocompatibility complex. By this presentation on the surface the peptides derived from the original antigen can be recognized by for instance helper T-lymphocytes, further activating the cellular immune response.

Thus Helper T-lymphocytes recognize exogenous antigens bound to major histocompatibility complex (MHC) class II molecules expressed by a variety of antigen presenting cells (APCs) such as B-lymphocytes, macrophages and dendritic cells (1). Compelling evidence indicates that newly synthesized α and β subunits of MHC class II in association with the invariant chain (I-chain) are transported to intracellular compartments before reaching the plasma membrane (2,3). In these compartments the 1-chain is degraded and MHC class II are potentially free to bind antigenic peptides arising from the degradation of antigens internalized by the APC (1, 4). We and others have shown that most of the intracellular MHC class II molecules reside in a Iysosome-like, MHC-class II-enriched compartment (MIIC) which contains characteristic membrane vesicles and concentrically arranged membrane sheets (5, 6, 7, 8, 9, 10). MIICs and the related CIIVs (11), likely represent the meeting point between MHC class II and antigenic peptides (8,12). Once loaded with peptide, MHC class II molecules are transferred to the cell surface via an unknown pathway for presentation to T-lymphocytes.

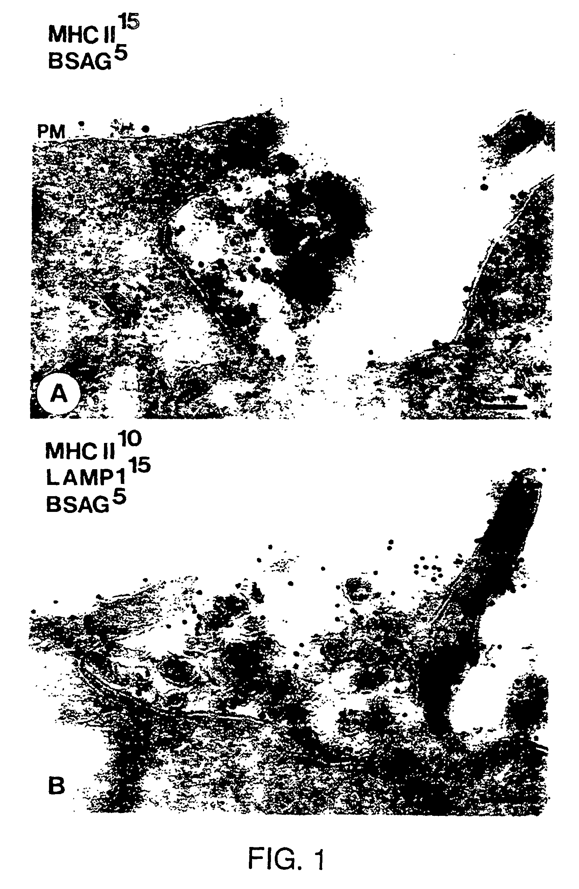

Electron microscopy of immunogold labeled ultra thin cryosections from several human B-lymphoblastoid cell lines revealed MIICs whose surrounding membrane was contiguous with the plasma membrane in an exocytotic fashion and showed extracellular vesicles reminiscent of those present in non-fused MIICs (Figure 1A and B). Similar secretion of vesicles, termed exosomes, has been described for reticulocytes (13). Exosomes from B cells immunolabeled for the Iysosomal membrane proteins LAMP1 (Figure 1 B) and CD63 (not shown) known to be expressed in MIICs (5, 6). Both LAMP1 and CD63 were absent from the rest of the plasma membrane. Scarce labeling for MHC class II was associated with the limiting membrane of the fused MIICs but MHC class II was enriched in the externalized exosomes (Figure 1A and B). To test the release of MIIC contents further, B cells were allowed to internalize 5 nm gold particles conjugated to Bovine Serum Albumin (BSAG), and were then washed and reincubated in the absence of BSAG. Exosomes associated with previously endocytosed BSAG began to appear in exocytotic profiles after 30 min of uptake (10 min pulse and 20 min chase) (Figure 1B) and were abundant after 50 min (10 min pulse and 40 min chase) (Figure 1A). We conclude that multivesicular MIICs of human B-cell lines can fuse with the plasma membrane thereby releasing MHC class II-rich exosomes into the extracellular milieu.

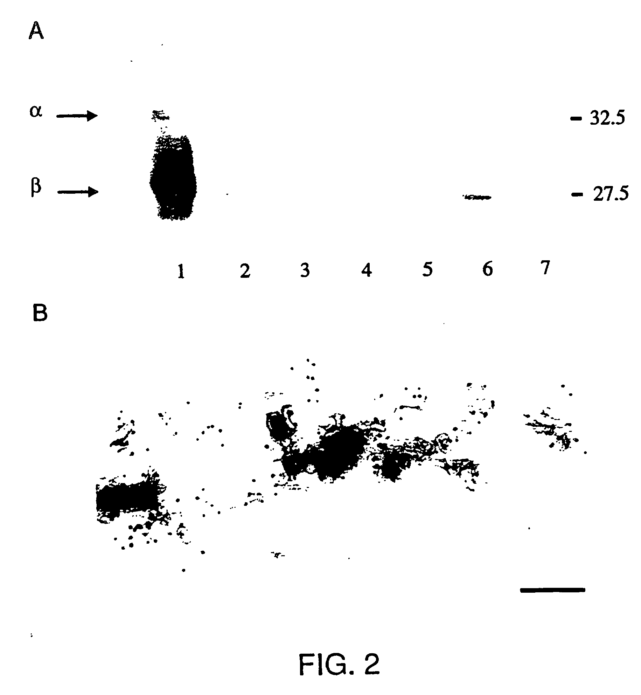

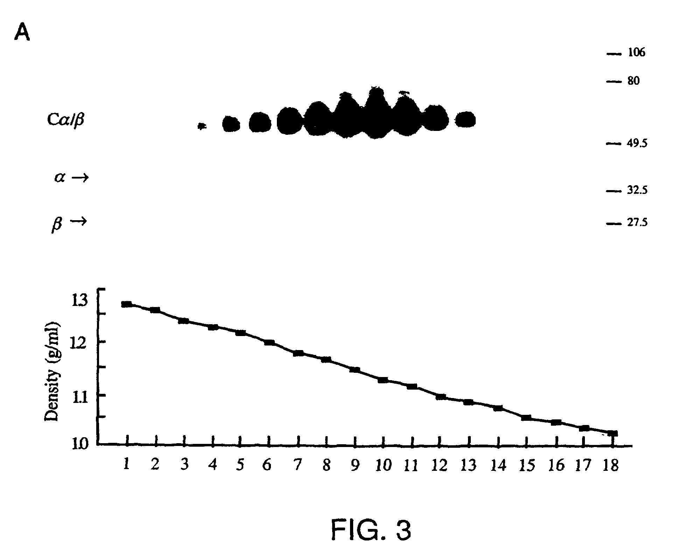

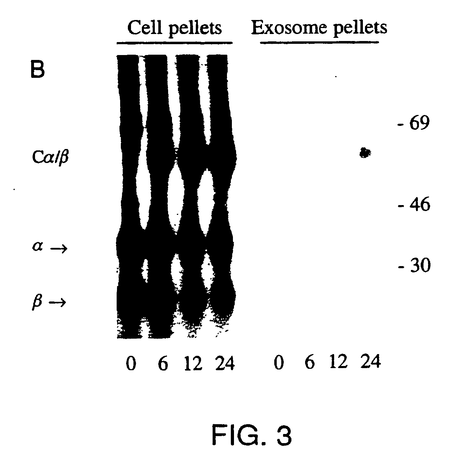

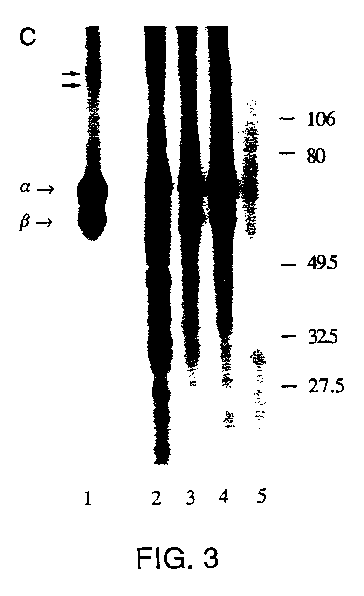

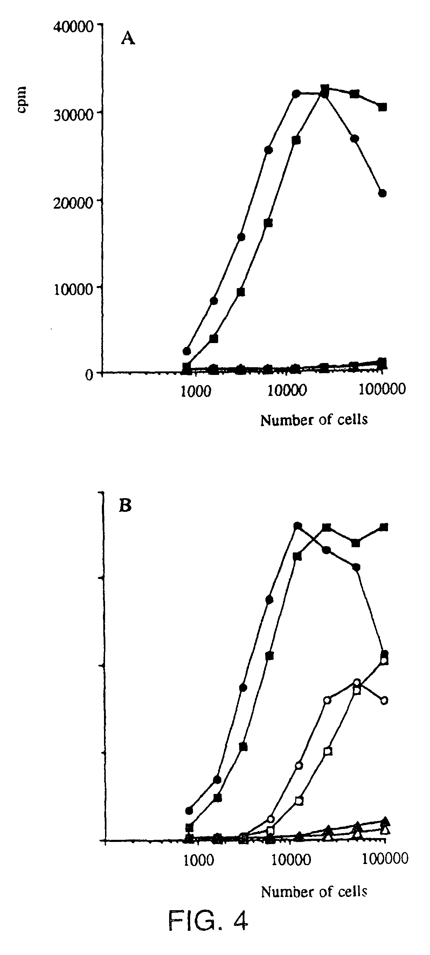

For a further characterization, exosomes were isolated from the culture media of the human B cell line RN by differential centrifugation (Figure 2). Pelleted membranes were analyzed by DS-PAGE and Western blotting. After removal of cells, the majority of MHC class II-containing membranes sediment at 70.000 g (Figure 2 A, lane 6). The 70.000 g pellets were composed of a homogeneous population of vesicles labeled for MHC class II (Figure 2 B). The vesicles were morphologically similar to those present in MIICs and in exocytotic profiles of sectioned cells (Figures 1 A and B): their size ranged from 60 to 80 nm. To obtain biochemical evidence that the secreted MHC class II is membrane bound, 70.000 g pellets were fractionated by floatation in linear sucrose gradients (14). Western blot analysis of the non-boiled and non-reduced gradient fractions showed that MHC class II molecules floated to an equilibrium density of 1.13 g/ml, confirming their association with membrane vesicles (Fig. A). MHC class II molecules recovered from the gradient fractions were predominantly in the SDS-stable, compact form indicating their stabilization by bound peptides (15). Together, these results show that the secreted MHC class II is associated with membrane vesicles and has bound peptides. To determine the kinetics and the extent to which newly synthesized MHC class II molecules are released into the medium, RN cells were metabolically pulse-labeled for 45 min. with [³⁵S]-methionine and chased for up to 24 hours in the absence of label (16). After pulse-labeling MHC class II was immunoprecipitated as SDS-unstable α-β-1-chains complexes (Fig.3 B, lane 0). At 6 hours of chase part of MHC class II molecules were converted to SDS-stable, α-β-peptide complexes consistent with the kinetics reported for other human B cell lines (2, 17). Recovery of [³⁵S]-compact MHC class II from pelleted exosomes started at 12 hours and amounted 10 + 4% (n=5) of the total newly synthesized MHC class II after 24 hours of chase. The relatively slow rate by which newly synthesized MHC class II was secreted into the medium suggests that insertion from the limiting membrane of MIICs into the plasma membrane during exocytosis is probably not the only pathway by which MHC class II molecules are delivered to the cell surface. To test the possibility that the vesicles recovered from the medium represented shed plasma membrane fragments or cell debris instead of exosomes, cells and exosome preparations were biotinilated and the patterns of the biotinilated proteins were studied by Western blotting with ¹²⁵I-Streptavidin (18). Figure 3 C reveals differential patterns of biotinilated proteins in exosomes and plasma membranes. Whereas plasma membranes show a broad spectrum of biotinilated proteins (Figure 3C, lane 2), two proteins are enriched in exosomes (Figure 3C, lanes 3 and 4). Immunoprecipitation of the biotinilated exosomal proteins with a monoclonal anti-class II antibody (19) identified these proteins as MHC class II (α and β subunits (Figure 3C, lane 1). Furthermore, the exosomes contain two minor bands at higher molecular weight which are not clearly detected in plasma membranes (Figure 3C, lanes 3 and 4). These proteins were also immunoprecipitated with the anti-class II antibody (Figure 3C, lane 1). To test the unlikely possibility that plasma membrane fragments eventually present in the 70.000 g pellets contributed to the enrichment of MHC class II in exosomes, biotinilated cells were homogenized and the homogenates were processed as the cell culture supernatants (18). Very low amount of membranes are pelleted at 70.000 g and these show a pattern of biotinilated proteins matching that of total plasma membrane, as expected (Figure 3C, lane 5). When the cells were metabolically labeled with [35S]-methionine for 45 min. and chased for up to 24 hours (16), the [³⁵S]-Transferrin receptor (TfR) ([³⁵S]-TfR) did not appear in exosomes at any chase time (data not shown). TfR is present at the plasma membrane of B cells but is absent from MIIC (8, 10). Together, these observations emphasize that exosomes are not derived from shed plasma membranes but represent an unique population of MHC class II- enriched membrane vesicles.

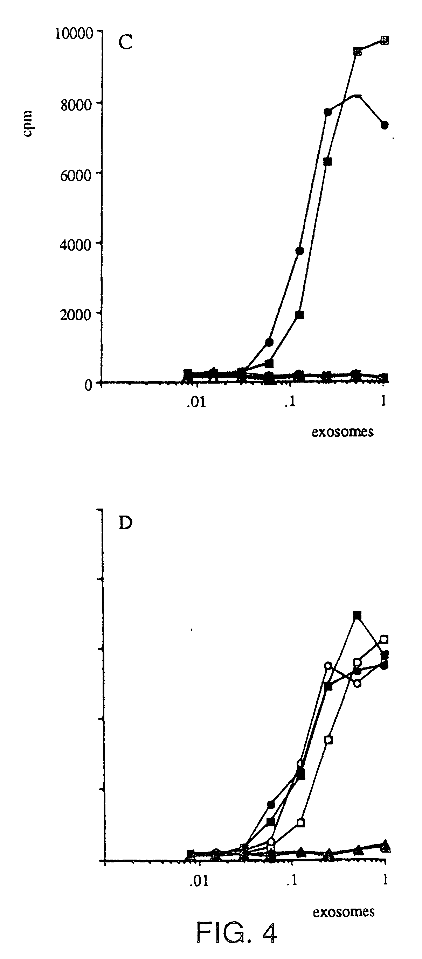

Since the luminal domain of MHC class II molecules is exposed at the outside of exosomes (20), exosomes may be able to present antigens to T cells. To test this hypothesis, isolated exosomes were allowed to bind peptide 418-427 from the model antigen HSP 65 of Mycobacterium Leprae. The exosome preparations were then added to the T cell clone 2F10 which recognizes this peptide in the context of HLADR15 (21). In a parallel experiment, RN cells were allowed to endocytose HSP65 protein continuously for 24 hrs, washed, and incubated in the absence of antigen for another 24 hrs (22). Both, exosomes incubated with antigenic peptide (Figures 4 A and C) and exosomes derived from cells that were pre-incubated with antigen (Figures 4 B and D) were able to induce a specific T cell response (23). A half maximal response was obtained with an amount of exosomes secreted by 3 x 10⁵ RN cells in 24 hours (Fig.4 D). In comparison 2x10⁴ intact RN cells were necessary to achieve the half maximal response (Fig.4 B, 24). The responses observed were DR restricted. Anti-HLA-DR antibody blocked T cell proliferation completely, whereas antiHLA-DP was ineffective (Figs 4 B and D). From these data we conclude that culture media of B cells provide for a source of MIIC-derived microvesicles (exosomes) that can induce T cell responses by themselves (25).

Exocytosis of MIIC vesicles by B-lymphocytes is reminiscent of the exocytosis of the vesicles contained in the cytolytic granules of cytotoxic T-lymphocytes (CTLs) (26). Both MIICs and cytolytic granules have Iysosomal characteristics and contain internal membranes. The internal vesicles of cytolytic granules are exocytosed by the CTLs upon CTL-target cell interaction and presumably have a role in the killing of target cells (26). Whether B-cell exosomes also have an extracellular role *in vivo* remains to be established. It has been suggested that follicular dendritic cells acquire MHC class II molecules released from surrounding B cells by an unknown mechanism (27). It is worth studying the possibility that exosomes serve as carriers of MHC class II-peptide complexes between different cells of the immune system. Whether physiological APCs like dendritic cells and macrophages generate exosomes has to be studied (28). However, secretion of Iysosomal contents by macrophages has been documented and macrophage tubular Iysosomes are rich in MHC class II and contain membrane vesicles (29). It can be speculated that in vivo, exosomes may function as transport vehicles for MHC class II-peptide complexes responsible for maintenance of long term T cell memory or T cell tolerance. Finally, since exosomes can easily be obtained and are capable of presenting antigens specifically and efficiently, it is worth exploring their usefulness as biological vehicles in immunotherapy.

It is therefore described an antigen presenting vesicle free from its natural surroundings obtainable from antigen presenting cells, such as B-cells, macrophages or dendritic cells, especially Langerhans cells of the epidermis.

These vesicles preferably will contain major histocompatiblility complex (MHC) I and/or II, most preferably loaded with a peptide derived from or corresponding to an antigen which can be processed by antigen presenting cells.

It has been tried before to produce similar vesicles synthetically, for instance in the form of liposomes, but these attempts have sofar not been successful. Now that we have surprisingly found that there are counterparts of said liposomes in nature, these counterparts can of course be used in any intended application of said liposomes.

The major advantage of the vesicles prepared according to the invention is of course that they will automatically comprise all the necessary elements for antigen presentation. Further analysis of the vesicles, once discovered will therefore result in a better understanding of which elements are essential for said presentation on said vesicles. It will then of course be possible to arrive at vesicles according to the invention in other ways then by isolation from cells. The invention therefor does encompass all antigen presenting vesicles which comprise the essential elements for presenting such antigens, regardless of the way they are produced or obtained.

One may for instance think of synthetically prepared liposomes, provided with at least biologically active parts of (recombinant) MHC I or II, optionally provided with processing agents for antigens to be presented in the context of said MHC. Of course cells which produce these vesicles can also be provided with recombinant MHC I or II encoding genes, so that the desired MHC's will be present on the eventually resulting vesicles, etc.

Although vesicles which present peptides in the context of MHC I or II are preferred, it is also very useful to produce vesicles which do have the MHC's on their surface, but without a peptide being present therein. These vesicles can then be loaded with desired peptides having the right binding motiv to fit in the respective MHC.

The first and perhaps foremost use of these vesicles that comes to mind is of course mimicking their role in nature, which is the presentation of peptides as antigens, for the stimulation of for instance T-cells. Thus the vesicles prepared according to the invention can be very suitably used in for instance vaccines. These vaccines can be designed to elicit an immune response against any proteinaceous substance which has peptide antigens that can be presented in the context of MHC.

The vaccines may of course comprise suitable adjuvants, if necessary, carriers, if necessary, ecxipients for administration, etc.

The vaccines can be used in the treatment or prophylaxis of many disorders, such as infections, immune disorders, malignancies, etc.

Very important applications will of course be the treatment or prophylaxis of AIDS, eliciting immuneresponses agains tumours and the like.

Another important application of the vesicles prepared according to the invention is that they may be used to induce tolerance to certain antigens, for instance by giving large doses of the vesicles orally.

Based on the description of the invention and specifically referring to the following experimental part illustrating the invention the person skilled in the art will be able to find further uses of the vesicles according to the invention.

### Legends to Figures:

### Figure 1:

MIICs are exocytotic compartments. T2-DR3 cells were incubated in the presence of 5 nm BSAG for 10 min., washed, chased for 40 min. and processed for cryoultramicrotomy as described (30). Ultrathin cryosections were immunolabeled with a rabbit polyclonal anti-class II antibody (5) and antibody binding sites were visualized with protein A conjugated to gold (PAG with sizes in nm indicated on the figures). MHC class II labeling is present at the limiting membrane of the exocytotic profile and on the exosomes. The profile also contains abundant re-externalized BSAG particles. PM: plasma membrane. B, RN cells were pulsed with BSAG for 10 min. and chased for 20 min. Ultrathin cryosections were double-immunolabeled with anti-class II antibody and with a monoclonal anti-LAMP1 antibody (31) as indicated. One of two neighboring profiles is shown, exocytotic profile containing BSAG and numerous exosomes labeled for MHC class II and LAMP1. Bars , 0.1 µm.

### Figure 2:

Isolation of exosomes from cell culture media. A, RN cells were washed by centrifugation and re-cultured in fresh medium for 2 days. Cell culture media (35 ml) containing 2-5 x10⁸ RN cells were centrifuged twice for 10 min. at 300 g (lane 1, first run; lane 2, second run). Lane 1 contains material from 0.6 x 10⁶ cells. Membranes in the culture medium from 2-5 x 10⁸ cells were pelleted by sequential centrifugation steps: twice at 1200 g (lane 3 and 4), and once at 10.000 g (lane 5), 70.000 g (lane 6) and 100.000 g (lane 7). The pellets were solubilized at 100°C under reducing conditions and analyzed by Western blotting using [¹²⁵l]-protein A. Per lane, samples equivalent to 1 x10⁶ cells were loaded. MHC class II α and β chains were recovered mainly from the cells (lane 1) and from the 70.000 g pellet (lane 6). B, whole mount electron microscopy of the 70.000 g pellet immunogold labeled for MHC class II. The 70.000 g pellet was resuspended in RPMI medium, adsorbed to Formvar-carbon coated EM grids, fixed with 0.5 % glutaraldehyde in 0.1 M phosphate buffer, immunolabeled with rabbit polyclonal anti-class II antibody and 10 nm PAG and stained using the method described for ultra-thin cryosections (30). The pellet is composed of 60-80 nm vesicles showing abundant MHC class II labeling. Bar, 0.2 µm

### Figure 3:

A, MHC class II present in the media are membrane bound. Membranes pelleted from culture media at 70.000g after differential ultracentrifugation were fractionated by floatation on sucrose gradients, and the non-boiled and non-reduced fractions analyzed by SDS-PAGE and Western blotting with the rabbit polyclonal anticlass II antibody (17). MHC class II molecules were recovered in fractions 5 to 12 corresponding to densities of 1.22-1.10 g/ml. The majority of MHC class II was in the SDS-stable compact form with a MW of 56-60 kD (Coc/β).

B, Release of newly synthesized MHC class II molecules. RN cells were pulse-labeled with [³⁵S] methionine for 45 min. (lane 0) followed by chases in the absence of label for 6, 12 and 24 hours. MHC class II molecules were immunoprecipitated from Iysates of the cells and pelleted exosomes with the monoclonal DA6.231 anti-class II antibody (18). Immunoprecipitated MHC class II molecules were dissociated from the sepharose beads at non-reducing conditions at room temperature and analyzed by SDS-PAGE and fluorography. After pulse-labeling (0), MHC class II immunoprecipitated from the cells as SDS-unstable complex of α-β-invariant chain. SDS-stable α-β dimers were recovered from the cells after 6 hours of chase and the signal increased thereafter. In the exosomes pellets SDS-stable αβ dimers started to appear at 12 hours. C, Exosomes and plasma membrane display different patterns of biotinilated proteins (18). In plasma membranes (lane 2) and experimentally produced remnants of plasma membranes (18) many biotinilated proteins are detected with ¹²⁵1Streptavidin (lane 5). In exosomes (lanes 3 and 4, show increasing concentrations of exosomes, respectively) two major proteins with a MW of 60-70 kD are detected. Lane 1 shows the immunoprecipitation of biotinilated class II α and β chains from exosomes Iysates. In these assay the higher electrophoretical mobility of α and β chains is due to their efficient binding to biotin. Two minor bands at a MW of 200-300 kD are detected in exosomes (lanes 1, 3 and 4, arrows) and are absent from the plasma membrane.

### Figure 4:

Presentation of HSP 65 antigen by HLA-DR15 positive RN B cells and exosomes to the CD4⁺ T cell clone 2F10 (22). Proliferative responses to naive cells (A), to cells pre-incubated with antigen (B), to exosomes derived from naive cells (C) and to exosomes derived from cells pre-incubated with antigen (D). The closed symbols show proliferation measurements after addition of HSP 65 derived peptide (418-427), the open symbols where peptide was not added. HLA-class II restriction was determined by adding 10 µg/ml anti-DR antibody (triangles), anti-DP (circles), or no antibody (squares). The exosomes at the highest concentration were derived from media of 1.6 x 10⁶ cells. All assays were performed in triplicate and results are expressed in cpm [³H]-thymidine incorporated into T cells. The SEM for triplicate cpm measurements was less then 10%. Results shown form a representative example of experiments performed in duplo.

### References and notes:

1. R. N. Germain, D. H. Margulies, *Annu. Rev. Immunol*. **11,** 403-450 (1993).
   P. Cresswell, *Annu. Rev. Immunol*. **12,** 259-293 (1994a).
   P. R. Wolf, H. L. Ploegh, *Annu. Rev. CellDev. Biol*. **11,** 267-306 (1995).
2. J. J. Neefjes, V. Stollorz, P. J. Peters, H. J. Geuze, H. L. Ploegh, *Cell* **61**, 171183 (1 990).
3. P. J. Benaroch, et al., *EMBO J.* **14**, 37-49 (1995).
4. P. A. Roche, P. Cresswell, *Proc. Natl. Acad. Sci. USA* **88,** 3150-3154 (1991).
5. P. J. Peters, J. J. Neefjes, V. Oorschot, H. L. Ploegh, H. J. Geuze, *Nature (Lond.)* **349,** 669-676 (1991).
6. J. M. Riberdy, R. R. Avva, H. J. Geuze, P. Cresswell, *J. CellBiol*. **125,** 1225-1237 (1994).
7. M. Kleijmeer, V. Oorschot, H. J. Geuze, *J. Invest. Dermatol*. **103,** 516-523 (1994).
8. M. A. West, J. M. Lucocq, C. Watts, *Nature (Lond.)* **369**, 147-151 (1994).
9. H. W. Nijman, et al., *J. Exp. Med*. **182,**163-174 (1995).
10. P. Peters, et al., *J. Exp. Med.* **In press**, (1995).
11. S. Amigorena, J. R. Drake, P. Webster, I. Mellman, *Nature (Lond.)* **369**, 113-120 (1994).
12. C. V. Harding, H. J. Geuze, *J. Immunol.* **151**, 3988-3998 (1993).
   A. Tulp, D. Verwoerd, B. Dobberstein, H. L. Ploegh, J. Pieters, *Nature (Lond.)* **369**, 120-126 (1994).
   Y. Qiu, X. Xu, A. Wandinger-Ness, D. P. Dalke, S. K. Pierce, *J. Cell Biol*. **125**, 595-605 (1994).
   A. Y. Rudensky, et al., *Immunity* **1**, 585-594 (1994).
13. C. Harding, J. Heuser, P. Stahl, *Eur. J. CellBiol*. **35**, 256-263 (1984).
   B. T. Pan, K. Teng, C. Wu, M. Adam, R. M. Johnstone, *J. Cell Biol*. **101,** 942-948 (1985).
14. The 70.000 g pellet obtained after differential centrifugation of the cell culture supernatants of RN B Iymphoblastoid cells was resuspended in 5 ml of 2.5 M sucrose, 20 mM Hepes/NaOH pH 7.2. A linear sucrose gradient (2 M-0.25 M sucrose, 20 mM Hepes- NaOH, pH 7.2) was layered over the exosome suspension in a SW27 tube (Beckman) and was centrifuged at 100.000 g for 15 hrs. Gradient fractions (18 x 2 ml) were collected from the bottom of the tube, diluted with 3 ml PBS and ultracentrifuged for 1 hr at 200.000 g using a SW50 rotor (Beckman). The pellets were solubilized at room temperature in SDS-sample buffer lackingmercaptoethanol and analyzed by SDS-PAGE and Western blotting using ¹²⁵ 1-Protein A.
15. R. N. Germain, L. R. Hendrix, *Nafure (Lond.)* **353,** 134-139 (1991). L. J. Stern, D. C. Wiley, *Cell* **68**, 465-477 (1992).
16. RN cells were pulsed for 45 min. with 50 Mbq/ml [35S]-methionine (Tran-Slabel, ICN, CA) and chased for different periods of time (5x107 cells per time point). After pulse-chase labeling, the cells were pelleted by centrifugation for 10 min. at 300 g. The supernatants were collected and centrifuged for 5 min. at 10.000 g and then for 30 min. at 200.000 g in a SW60 rotor (Beckman). Cells and the 200.000 g pellets were Iysed and MHC class II and TfR were immunoprecipitated from equal samples of the Iysates. TfR was immunoprecipitated as described previously [W. Stoorvogel, H. J. Geuze, J. M. Griffith, A. L. Schwartz, G. J. Strous, J. *CellBiol*. **108**, 2137-2148 (1989)]. MHC class II was quantitated using a Phosphoimager.
17. J. J. Neefjes, H. L. Ploegh, *EMBO J.* **11**, 411-416 (1992).
18. RN cells (2 x 10⁸) were washed 3 times with ice cold PBS and incubated for 30 min. at 0°C with 1mg/ml Sulfo-NHS-biotin (Pierce). Biotin was quenched for 30 min. with 50 mM NH4 Cl. After washing with ice cold PBS, half of the cells were solubilized in SDS-sample buffer supplemented with β-mercaptoethanol. The remaining biotinilated cells were homogenized. The homogenates were centrifuged and ultracentrifuged identically to the cell culture supernatants and the 70.000 g pellets solubilized in SDS-sample buffer supplemented with β-mercaptoethanol (control for plasma membrane remnants). Exosome preparations (70.000 g pellets of cell culture media from 2 x 10 8 cells) were biotinilated as described above and solubilized in SDS-sample buffer supplemented with β-mercaptoethanol. MHC class II was immunoprecipitated from a sample of biotinilated exosomes with the monoclonal anti-class II antibody DA6.231 (19). The biotinilated cell membranes, biotinilated exosomes and immunoprecipitated MHC class II were analyzed by SDS-PAGE and Western blotting with ¹²⁵l-Streptavidin.
19. K. Guy, V. Van Heyningen, B. B. Cohen, D. L. Deane, C. M. Steel, *Eur. J.* / *mmunol*. **12,** 942-948 (1982).
20. The internal MIIC vesicles are formed by inward budding of the limiting membrane of MIICs (see figures 16 and 17 in reference [6 ] similar to the process described for multivesicular bodies in other cell types [B. van Deurs, P. K. Holm, L. Kayser, K. Sandvig, S. H. Hansen, *Eur. J. Cell Biol*. **61**, 208-224 (1993)].
21. T. H. M. Ottenhof, et al., *Nature (Lond.)* **319**, 66-68 (1986).
   J. B. A. G. Haanen, et al., *J. Exp. Med.* **174**, 583-592 (1991).
22. The EBV-B cell lines RN (HLA-DR 15+) and JY (HLA-DR15-) were incubated in the presence or absence of purified HSP 65 protein from Mycobacterium Leprae (50µg/ml) [J.E.R. Thole, et al., *Microbial Pathogenesis* **4**, 71-83 (1988)] for 4 hr in 10 ml serum free RPMI at 2 x 10⁶ cells /ml, followed by the addition of 30 ml RPMI supplemented with 10% fetal calf serum (FCS) for 20 hr at 37°C. The cells were then washed to remove free antigen and incubated further for 24 hrs in RPMI/10% FCS medium at 37°C. Exosomes were prepared by differential centrifugation (Figure 2) and the efficiency of HSP 65 antigen presentation was measured by culturing 10.000 cells of the T cell clone 2F10 with irradiated (6.000 rad) EBV cells. B cells or exosomes resuspended in 100 µl IMDM /10% pooled human serum were added to the T cell clone (50 µl IMDM /10% pooled human serum per well) in 96 well flatbottom microtitre plates (Costar, The Netherlands) for 4 days at 37°C, 5% C02 in humidified air. When indicated, 5 µg/ml of HLA-DR15 restricted epitope of HSP65 (peptide 418-427) was added to the exosomes. Sixteen hours before termination 0.5 µCi of [³H]-thymidine was added to the wells. The cells were then harvested on glass fiber filters using an automatic cell harvester and the [³H]-thymidine incorporation into cell DNA was determined by liquid scintillation counting. The results are expressed as the mean of triplicate measurements).
23. As a control, exosomes were prepared from culture media of an equivalent amount of DR15-negative JY cells that have been incubated or not with antigen. JY cells secreted an equivalent amount of exosomes but these were ineffective in stimulating T cell proliferation.
24. From these data exosomes appear to be 16 times less efficient in antigen presentation. However, in antigen presentation assays contact between B and T cells may be more efficient due to sedimentation of cells.
25. Exosomes isolated from the culture medium of the murine B cell line TA3 (1-E^{k+}) incubated in the presence a RNase-derived peptide (aa 90-105) were also capable of stimulating IL2 secretion by WA.23 cells.
26. P. Peters, H. J. Geuze, H. A. van der Donk, J. Borst, *Immunol. Today* **11**, 28-32 (1990)
   P. J. Peters, et al., J. *Exp. Med.* **173**, 1099-1109 (1991 b).
27. D. Gray, M. Kosco, B. Stockinger, *Int. Immunol.* **3**, 141-148 (1991).
28. A number of studies documented the presence of intact MHC class II molecules in 100.000 g fractions from B cell culture media and their association of with membrane lipids [S. G. Emerson, R. E. Cone, J. *Immunol*. **122**, 892-899 (1979);
   D. H. Sachs, P. Kiszkiss, K. J. Kim, J. *Immunol*. **124**, 2130-2136 (1980); S. G. Emerson, R. E. Cone, J. *Immunol*. **127**, 482-486 (1981)]. Our present observations shed new light on these data and suggest that the released MHC class II molecules were likely derived from secreted exosomes.
29. C. V. Harding, H. J. Geuze, J. *CellBiol*. **119,** 531-542 (1992).
30. J. W. Slot, H. J. Geuze, S. Gigengack, G. E. Lienhard, D. James, J. *Cell Biol*. **113,** 123- 135 (1991).
   W. Liou, J. W. Slot, *Proc. Int. Conf.Electr.Microsc*. **13,** 253-254 (1994).
31. S. R. Carlsson, J. Roth, F. Piller, M. Fukuda, J. *Biol.Chem*. **263**,18911 (1988).

## Claims

1. A method for the preparation of an antigen-presenting vesicle, the method comprising the steps of differential centrifugation of membrane fractions of a B-lymphocyte culture supernatant and recovery of a fraction containing antigen-presenting vesicles, wherein the vesicles comprise a class I or class II major histocompatibility complex.

2. A method according to claim 1, wherein the fraction is a 70 000 x g pellet obtained by differential centrifugation.

3. A method according to claim 1 or 2, wherein the vesicle additionally comprises processed antigens.

4. A method according to claim 3, wherein said processed antigens are bound to said class I or class II major histocompatibility complex.

5. A vesicle obtainable by a method according to any one of claims 1-4 for use as a medicament.

6. A vaccine composition comprising a vesicle of claim 5 and an adjuvant or carrier.

7. Use of a vesicle of claim 5 in the preparation of a medicament for the treatment or prophylaxis of an immune disorder or an infection.

## Patentansprüche

1. Verfahren für die Zubereitung eines Antigen-präsentierenden Vesikels, wobei das Verfahren die Schritte einer Differenzialzentrifugierung von Membranfraktionen eines B-Lymphozytenkulturüberstands und Rückgewinnung einer Fraktion, die Antigenpräsentierende Vesikel enthält, umfasst, wobei die Vesikel einen Klasse I- oder Klasse II-Haupthistokompatibilitätskomplex umfassen.

2. Verfahren gemäß Anspruch 1, wobei die Fraktion ein 70.000 x g-Pellet ist, das durch Differenzialzentrifugierung gewonnen wurde.

3. Verfahren gemäß Anspruch 1 oder 2, wobei das Vesikel zusätzlich prozessierte Antigene umfasst.

4. Verfahren gemäß Anspruch 3, wobei die prozessierten Antigene an den Klasse I- oder Klasse II-Haupthistokompatibilitätskomplex gebunden sind.

5. Vesikel, erhältlich durch ein Verfahren gemäß einem der Ansprüche 1-4, zur Verwendung als ein Medikament.

6. Vakzinzusammensetzung, umfassend ein Vesikel gemäß Anspruch 5 und ein Adjuvans oder einen Träger.

7. Verwendung eines Vesikels gemäß Anspruch 5 für die Zubereitung eines Medikaments zur Behandlung oder Prophylaxe einer Immunerkrankung oder Infektion.

## Revendications

1. Méthode de préparation d'une vésicule présentatrice d'antigène, la méthode comprenant les étapes de centrifugation différentielle de fractions membranaires d'un surnageant de culture de lymphocytes B et de récupération d'une fraction contenant des vésicules présentatrices d'antigènes, dans laquelle les vésicules comprennent un complexe majeur d'histocompatibilité de classe I ou classe II.

2. Méthode selon la revendication 1, dans laquelle la fraction est un culot de 70 000 x g obtenu par centrifugation différentielle.

3. Méthode selon la revendication 1 ou 2, dans laquelle la vésicule comprend en outre des antigènes transformés.

4. Méthode selon la revendication 3, dans laquelle lesdits antigènes transformés sont liés audit complexe majeur d'histocompatibilité de classe I ou classe II.

5. Vésicule susceptible d'être obtenue par une méthode selon l'une quelconque des revendications 1-4 pour une utilisation comme médicament.

6. Composition vaccinale comprenant une vésicule selon la revendication 5 et un adjuvant ou support.

7. Utilisation d'une vésicule selon la revendication 5 dans la préparation d'un médicament pour le traitement ou la prophylaxie d'un désordre immunitaire ou d'une infection.
